## Europäisches Patentamt

(19) **European Patent Office**

Office européen des brevets

(11) Publication number: **0 182 756**
**B1**

(12) **EUROPEAN PATENT SPECIFICATION**

(45) Date of publication of patent specification: **30.01.91**

(51) Int. Cl.⁵: **A 61 K 7/06, A 61 K 7/48**

(21) Application number: **85830288.8**

(22) Date of filing: **20.11.85**

(54) Dermotropic cosmetic compositions.

(30) Priority: **20.11.84 IT 2367684**

(43) Date of publication of application:
**28.05.86 Bulletin 86/22**

(45) Publication of the grant of the patent:
**30.01.91 Bulletin 91/05**

(84) Designated Contracting States:
**AT BE CH DE FR GB LI LU NL**

(56) References cited:
**EP-A-0 084 157**
**FR-A-1 186 146**
**FR-A-1 489 740**
**FR-A-2 036 453**
**LU-A- 56 945**

**CHEMICAL ABSTRACTS, vol. 96, no. 16, April 1982, page 447, abstract no 129594h, Columbus, Ohio, US; & JP-A-81 158 706 (ICHIMARU CO., LTD) 07-12-1981**
**Idem**

(73) Proprietor: **FARMAKA S.r.l.**
**Via Vetreria, 1**
**Grandate (Como) (IT)**

(72) Inventor: **Casero, Riccardo**
**Via Rovascino, 21**
**Lipomo (Como) (IT)**
Inventor: **Casero, Alessandro**
**Via Imbonati, 11**
**Lipomo (Como) (IT)**

(74) Representative: **Aimi, Luciano et al**
**c/o Società Italiana Brevetti S.p.A. Via Carducci 8**
**I-20123 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

The present invention relates to new cosmetic compositions endowed with entrophic and moisturizing properties to be applied to skin for preventing or repairing ageing thereof. In particular, the present invention relates to new cosmetic compositions suitable for strengthening hair, preventing its falling out and stimulating hair growth.

It is known that cutis, due to the particular functions to which it is devoted, is structured in a particular way and is substantially comprised of two superimposed layers, namely the underlying derma and the epidermis forming the external surface of cutis.

As epidermis is devoid of blood vessels, even if it has to carry out many of the most remarkable functions of the skin, such as the protective, secretive, thermoregulating, respiratory functions, and so on, metabolic impairments often occur, affecting its appearance and jeopardising its functions, also in a serious manner.

It is also known that hair, as the essential part of the human piliferous apparatus, is planted in epidermis, so that it also shares in a very considerable way its vicissitudes and deficiencies, and therefore falling out of hair is a more and more frequent phenomenon both in men and in women, also in not typically pathological situations. The multifactorial aetiogenesis of such a falling out, having a developing course (stress, atmospheric pollution, emotional factors, washing with strongly detergent shampoos, hormonal factors, etc) is always connectable with a morphological alteration of the connective tissue surrounding the hair bulb which is determined, inter alia, by the decrease of specific acidic mucopolysaccharides (heparin, hyaluronic acid, A, B, C, chondroitinsulphates) in the connective tissue itself, with dehydration, consequent ageing of the tissue and dekeratinisation, together with fragility and poor elasticity of hair, and then sclerosis and death of the hair bulb.

In order to prevent ageing of cutis and, particularly, falling out of hair, different criteria have been followed till now, such as the local use of degreasing, rubefacient and vasodilating agents, polyvitaminic compositions and vegetable extracts of different kinds, as well as mucopolysaccharide extracts with unspecified compositions and activity (with particular reference to the anticoagulant and lipasemic activities, which are closely correlated and interdependent activities) alone or in combination.

However, none of these compounds has ever faced in a rational way, and consequently solved, the problem of physiological hydration of cutis and of the tissue surrounding the hair bulb, which, as above mentioned, is an essential condition for the vitality of the bulb itself and of hair.

Even if the use of sulphomucopolysaccharides, which notoriously possess hydrophilic properties, is already known, it is likewise known that they usually are substantially not absorbed through cutis.

For example EP—A—0 084 157 describes a cosmetic composition for chappy skin comprising conventional cosmetic bases or vehicles blended with a human umbilical cord or cockscomb extract. The composition is tested for water retention by dipping thereinto cut epithelia (i.e. stratum corneum) from rats. No effect on hair growth is mentioned.

The applicant has now discovered that it is possible to provoke and facilitate the cutaneous absorption of mucopolysaccharides by mixing them with an umbilical cord extract, free from chondroitinsulphates and rich in hyaluronic acid, obtained under particular conditions.

The purpose of this invention is therefore to produce a preparation substantially based on mucopolysaccharides (MPS) and extract of umbilical cord (ECO), able to promote, by topical application, the hydration and trophism of skin, and particularly of hair.

The present invention is based on the ascertainment that an extract of umbilical cord, besides possessing its own high hydrophilic and hydrating properties, allows the absorption of the essential MPS by skin, owing to the presence of the peptide chains of proteohyaluronic acid therein contained, which, by promoting an increase in the peripheral blood microcirculation, allows an effective absorption of the aforesaid substances. A further problem solved by this invention is that of choosing the acidic mucopolysaccharides which have to be supplied to skin and must be such as to correct the typical deficiency of these substances occurring in concommitance with falling out of hair and ageing of cutis. The MPSs chosen for topic application have furthermore to act, through their clarifying (i.e. anticoagulating/normolipemising) activity, as regulators for the sebaceous secretion which is a contributory cause of hair weakening and of sclerosis of hair bulb, all causes bringing to the common baldness or androgenetical or seborrhoical alopecia.

The complex of extractive acidic mucopolysaccharides of animal origin according to this invention is rich in chondroitinsulphates and heparin-like substances and is characterised by the following essential analytical parameters:

# EP 0 182 756 B1

| pH in 5% aqueous solution | 5 to 7.5 |
|---|---|
| rotatory power | 0 to +15° |
| proteins | absent |
| total N | $\leqq 4.5\%$ |
| S | $\geqq 6\%$ |
| inorganic P | $\leqq 0.5\%$ |
| hexuronic acids | $\leqq 20\%$ |
| hexosamines | $\leqq 20\%$ |
| anticoagulating activity | 40—50/mg I.U. USP, XX |

The extract of umbilical cord to be mixed with the MPSs according to this invention is an aqueous extract of human or animal umbilical cord containing, on average, 5 to 7 g/litre of hyaluronic acid, principally in the form of proteohyaluronic acid (proteoglycane), and containing, moreover, C unsulphonated chondroitin. The hyaluronic acid, as contained in this extract, shows a molecular weight between 2.5 and 3 million.

An aqueous extract of human umbilical cord shows, for instance, the following essential analytical features:

viscous, transparent, slightly yellowish solution

| Ph | 5 to 7 |
|---|---|
| viscosity at 30°C | $1.10^{-1}$ cm$^2$.s$^{-1}$ to $2,5.10^{-1}$ cm$^2$.s$^{-1}$ (10 to 25 centistokes) |
| residue at evaporation | 2 to 3% |
| hyaluronic acid titre | 500 to 700 mg/dl |
| ashes | 0.1 to 0.5% |
| S | absent |
| proteins | 0.5 to 1.0% |
| total N | 0.15 to 0.30% |
| aminic N | 0.10 to 0.20% |
| hexosamines | 0.010 to 0.050% |

The process for obtaining the extract of umbilical cord according to the invention is as follows: A certain quantity of umbilical cord is washed with water, crumbled by means of a triturator and conveyed into a mixer, to which a volume of water twice as much as that of the crumbled tissue is added. The obtained aqueous mush is milled and the whole is well mixed at low temperature. The mixed suspension is then filtered and the filtrate is concentrated at reduced pressure and low temperature.

4.5% of methyl-para-hydroxybenzoate and 0.5% of propyl-parahydroxybenzoate are separately dissolved in a volume of ethanol equivalent to 5% by volume of the concentrated solution. The two solutions are mixed and filtered, and the filtrate is then sterilized by filtration through a membrane.

The sterilized solution is at last closed into hermetic vessels for subsequent use.

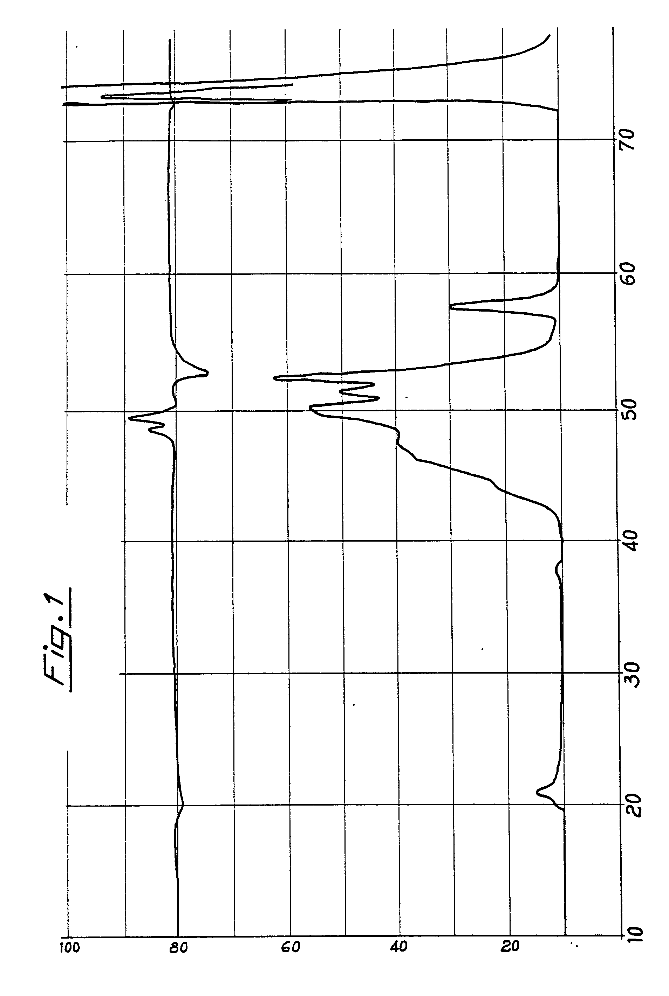

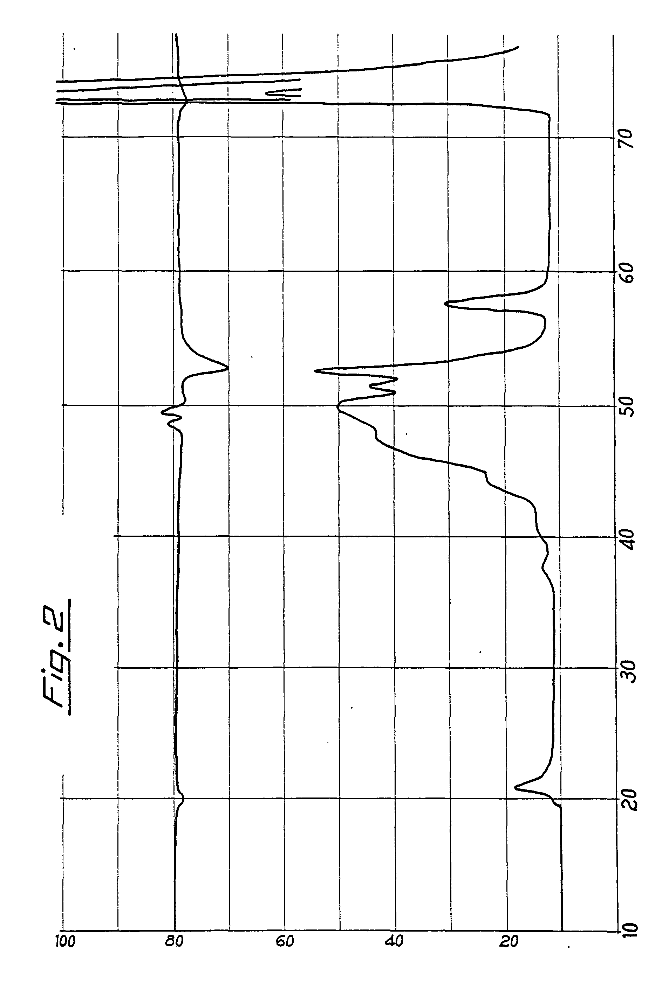

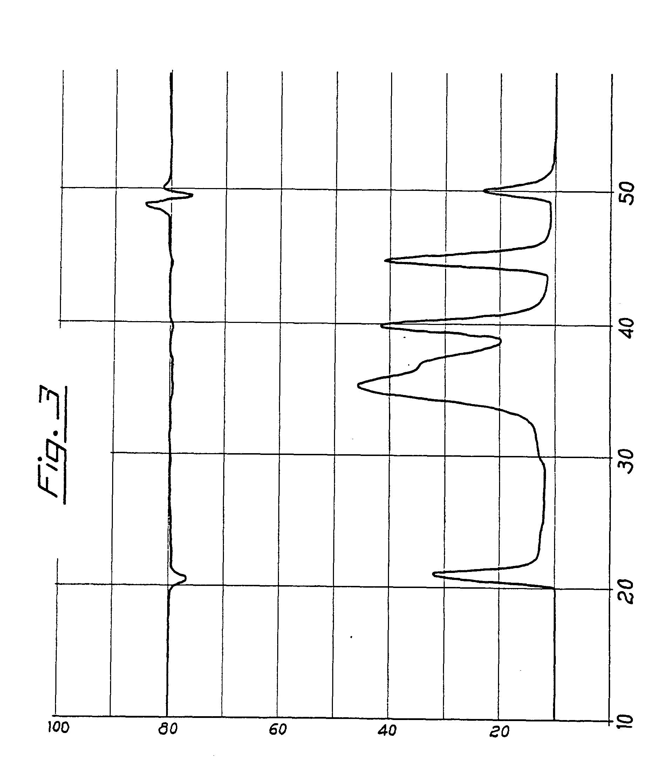

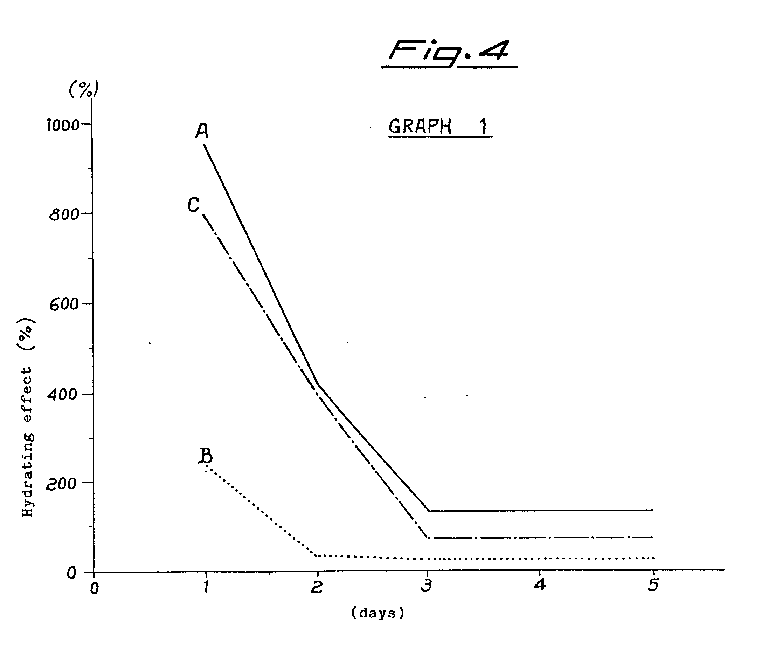

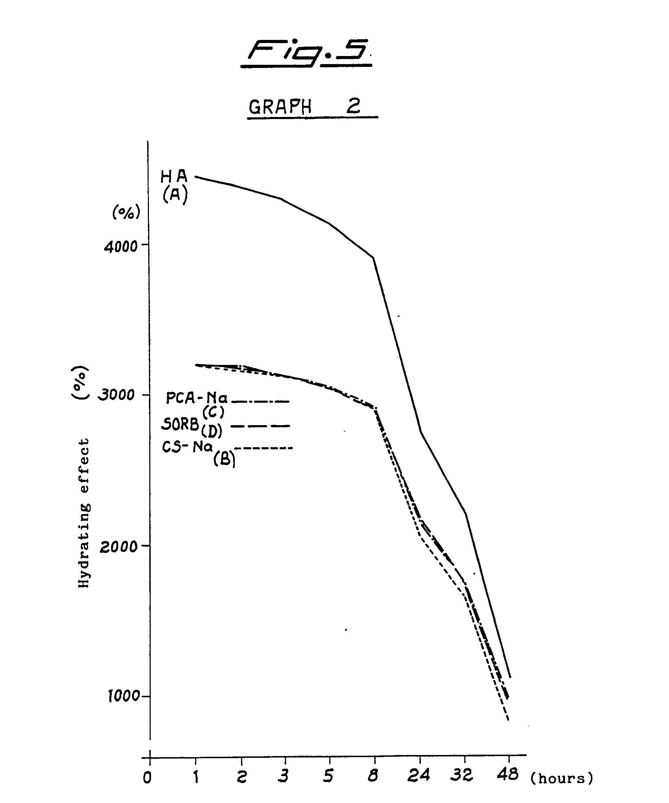

The enclosed figures 1 and 2 shows the graphs obtained from two samples of extract of umbilical cord according to the present invention by high speed gas-chromatography with a mobile phase consisting of an aqueous solution of KCl 0.1M buffered at pH 7.0 with a solution of phosphate 1/15M; figure 3 shows the gas-chromatographic graph obtained from a standard solution containing blue dextran 2000, gammaglobulin, lactoalbumin, eggalbumin, myoglobulin and alanine in the same operational conditions.

The following examples, illustrate the particular properties of moisture retention shown by extracts of human umbilical cord which have been used in the formulations according to the present invention, in comparison with similar properties shown by other known substances having similar characteristics.

3

### Example 1

The tests were carried out by using the following materials:

A) Extract of human umbilical cord (HA) with residue at evaporation = 2.23%

B) Solution of sodium chondroitinsulphate (CS-Na) with residue at evaporation = 3.0%

C) Solution of sodium pyrrolidonecarboxylate (PCA-Na) with residue at evaporation = 3.0% Carefully weighed samples of 20 g of A, B and C, respectively placed in glass capsules having an inside diameter of 85 mm and a depth of 20 mm, were put into a drier adjusted at 30°C and 80% of relative humidity. The so prepared samples were then weighed every 24 hours so as to evaluate the undergone weight changes.

The hydrating effect was obtained on the basis of the following ratio:

$$\text{Hydrating effect (\%)} = \frac{\text{Residual water}}{\text{Residue at evaporation}} \times 100$$

The obtained results are shown in the following Table 1 and schematically illustrated in the relevant graph 1.

### TABLE 1

| Sample | | Days | | | | |
|---|---|---|---|---|---|---|
| | | 1 | 2 | 3 | 4 | 5 |
| A | Residue at evaporation | 446 mg | 446 mg | 446 mg | 446 mg | 446 mg |
| | Residual water | 4254 mg | 1854 mg | 554 mg | 554 mg | 554 mg |
| | Hydrating effect (%) | 953.8% | 415.7% | 124.2% | 124.2% | 124.2% |
| B | Residue at evaporation | 600 mg | 600 mg | 600 mg | 600 mg | 600 mg |
| | Residual water | 1400 mg | 200 mg | 100 mg | 100 mg | 100 mg |
| | Hydrating effect (%) | 233.3% | 33.3% | 16.6% | 16.6% | 16.6% |
| C | Residue at evaporation | 600 mg | 600 mg | 600 mg | 600 mg | 600 mg |
| | Residual water | 4800 mg | 2400 mg | 500 mg | 447 mg | 363 mg |
| | Hydrating effect (%) | 800.0% | 400.0% | 83.3% | 74.5% | 60.5% |

### Example 2

Tests similar to those of Example 1 were carried out on the same substances A, B and C, and, furthermore, on the following substance:

D) A solution of sorbite (SORB) with residue at evaporation = 3.0%

Some samples of 1.0 g of substances A, B, C and D, respectively collected into glass vessels with a diameter of 30 mm and depth of 30 mm, were put into a drier in the presence of a saturated solution of $K_2CO_3$. The samples so treated were then weighed after 1, 2, 3, 5, 8, 24, 32 and 48 hours so as to record the undergone weight changes.

The hydrating effect was calculated on the basis of the ratio reported in Example 1. The obtained results are shown in the following Table 2 and schematically illustrated in the corresponding graph 2.

4

TABLE 2

| Time | Sample ⟶ | A | B | C | D |
|---|---|---|---|---|---|
| 0 hours | Residual water (mg) | 912.9 | 931.5 | 989.7 | 986.4 |
| | Residue at evaporation (mg) | 20.25 | 28.81 | 30.61 | 30.51 |
| 1 hours | Residual water (mg) | 901.2 | 921.7 | 980.7 | 975.8 |
| | Hydrating effect (%) | 4450.5 | 3199.3 | 3203.9 | 3198.6 |
| 2 hours | Residual water (mg) | 885.2 | 909.4 | 969.8 | 967.4 |
| | Hydrating effect (%) | 4371.0 | 3156.6 | 3168.3 | 3171.1 |
| 3 hours | Residual water (mg) | 868.5 | 897.3 | 957.6 | 952.5 |
| | Hydrating effect (%) | 4288.6 | 3114.6 | 3128.5 | 3122.3 |
| 5 hours | Residual water (mg) | 836.9 | 872.6 | 933.0 | 927.4 |
| | Hydrating effect (%) | 4132.5 | 3028.9 | 3048.1 | 3039.9 |
| 8 hours | Residual water (mg) | 788.1 | 830.4 | 892.3 | 883.9 |
| | Hydrating effect (%) | 3892.0 | 2882.4 | 2915.1 | 2897.3 |
| 24 hours | Residual water (mg) | 555.6 | 591.5 | 657.4 | 658.1 |
| | Hydrating effect (%) | 2743.4 | 2053.2 | 2147.7 | 2157.2 |
| 32 hours | Residual water (mg) | 448.2 | 476.5 | 537.6 | 531.0 |
| | Hydrating effect (%) | 2213.0 | 1654.0 | 1756.3 | 1740.6 |
| 48 hours | Residual water (mg) | 225.4 | 244.5 | 305.7 | 295.0 |
| | Hydrating effect (%) | 1112.8 | 848.7 | 998.7 | 967.0 |

From the above shown and illustrated data it is clear that the extract of human umbilical cord, used according to this invention, shows water retention effects which are decidedly higher than those of substances already known for their properties of absorbing and retaining water.

The mixtures of umbilical cord extract and mucopolysaccharide complex according to this invention normally contain also other active elements, adjuvants or additives, such as for instance vasodilators, preservatives, wetting agents, fungicidal agents, surfactants, bactericides, vitamins, pH regulating agents, etc.

Some examples of presently preferred formulations, according to this invention are hereinafter given in the form of numerical data.

### Example 3

Lotion for treating the scalp

| | |
|---|---|
| Extract of umbilical cord | mg 120.00 |
| Mucopolysaccharide complex | mg 8.58 |
| Tetrahydrofurfuryl-nicotinate | mg 4.50 |
| Propylenglycol | mg 0.20 |
| Methylparaben | mg 8.10 |
| Propylparaben | mg 3.90 |
| Sorbic acid | mg 6.00 |
| Sodium pantothenate | mg 6.00 |
| Biotin | mg 0.15 |
| Ethanol | mg 0.44 |
| Perfume | q.s. |
| Distilled water | q.s. to ml 6.00 |

The above given formulation is prepared in the form of vials.

### Example 4

Face moisturizing lotion

| | |
|---|---|
| Mucopolysaccharidic complex | g 0.3 |
| Extract of umbilical cord | g 2.0 |
| dl-α-tocopherol | g 0.5 |
| Ethanol | g 13.0 |
| Glycerine | g 2.0 |
| Propyleneglycol | g 5.0 |
| Polyoxyethylene 20 Sorbitan monolaurate 20 moles of ethylene oxide | g 2.0 |
| Polyethyleneglycol monostearate | g 1.0 |
| Methylparaben | g 0.2 |
| Propylbaren | g 0.1 |
| Perfume | q.s. |
| Purified water | q.s. to g 100 |

EP 0 182 756 B1

Example 5

Face moisturizing cream

| | |
|---|---|
| Mucopolysaccharide complex | g 2.0 |
| Potassium glycerinizate | g 0.1 |
| dl-α-tocopherol acetate | g 0.05 |
| Extract of umbilical cord | g 3.0 |
| Squalene | g 8.0 |
| White vaseline | g 3.0 |
| Amitel LG-OD (di-2-octyldodecyl-N-lauroyl-L-glutamate) | g 5.0 |
| White wax | g 1.0 |
| Glyceryl-monostearate | g 1.0 |
| Propyleneglycol monostearate | g 4.0 |
| Sorbitane monostearate | g 4.0 |
| N-acyl-L-sodium-glutamate | g 0.2 |
| Solution of sorbitol (70%) | g 5.0 |
| Glycerin | g 2.0 |
| Stearic acid | g 2.0 |
| Silicone resin | g 0.5 |
| Propylparaben | g 0.1 |
| Methylparaben | g 0.2 |
| Perfume | g 0.05 |
| Purified water | q.s. to g 100 |

7

## Example 6

Anticellulitis cream

| | |
|---|---|
| Stearic acid | g 15.0 |
| Cetyl palmitate | g 8.0 |
| anhydrous lanolin | g 4.0 |
| n-propyl p-oxybenzoate | g 0.1 |
| Tween 80 (Polysorbate 80 or Polyoxyethylene Sorbitan monolaurate) | g 2.5 |
| 1.2-propylene glycol | g 5.0 |
| Triethanolamine | g 2.0 |
| Methyl p-oxybenzoate | g 0.2 |
| Mucopolysaccharide complex | g 0.3 |
| Escin | g 1.0 |
| Tetrahydrofurfuryl-nicotinate | g 0.05 |
| Distilled water | g 61.0 |
| Extract of umbilical cord | g 1.0 |
| Perfume | 3 drops |

The weight ratio of the umbilical cord extract to the mucopolysaccharide complex contained in the formulations according to this invention ranges from 1:1 to 15:1.

The cosmetic formulations according to this invention are characterized by a number of properties which may be resumed as follows:

Remarkable hydrating effect on cutis, which avoids the dehydration of the horny layer, retains water, prevents dandruff, reduces statical electricity of hair making easier the action of the comb, increases hair elasticity making hair softer; ability to form membranes having greater strength and elasticity than those obtained with other products as polyvinyl alcohol, polyvinyl pyrrolidone or gelatine, and that increases the hydrating effect and facilitates the action of the comb; improvement of the subcutaneous microcirculation through topical peripheral vasodilation and permanent action on the connective tissue; antibacterial activity due to a phenomenon of anionic repulsion which is typical of the extract of umbilical cord, with consequent hindrance to the dandruff formation.

The sum of these properties and characteristics allows therefore to extend the use of the formulations according to this invention to all the morphological alterations of the subcutaneous connective tissue, which are characteristic of premature ageing of skin in different cutaneous districts. Therefore, both active agents, conveniently chosen and mixed according to this invention (extract of umbilical cord and mucopolysaccharide complex) and rightly conveyed and combined with other substances, differentiated according to the use and the concerned district, may be usefully employed in the different forms of chronic alopecia and particularly in the androgenetic and seborrhoic alopecia, in the treatment of dry and arid skins in order to avoid the formation of wrinkles or to attenuate the already formed wrinkles, in the topical treatment of cellulitis, owing to the vasodilating action and metabolic activation which favours the elimination of adipose cushions, as well as in every other non-pathological skin unaesthetisms as they are sustained, in most cases, by an insufficient water content of derm.

## Claims for the Contracting States: BE CH DE FR GB LI LU NL

1. Dermotropic cosmetic compositions comprising an aqueous extract of umbilical cord, characterized in that said extract of umbilical cord is free from sulphur, has a pH from 5 to 7, a kinematic viscosity at 30°C of $1.10^{-1}$ cm$^2$.s$^{-1}$ to $2,5.10^{-1}$ cm$^2$.s$^{-1}$ (between 10 and 25 centistokes), a hyaluronic acid titre ranging from 500 to 700 mg/dl and an ash content between 0,1 and 0,5%, and is mixed in proportions ranging from 1:1 to 15:1 with further mucopolysaccharide complexes not containing proteins, having a pH from 5 to 7,5 in 5% aqueous solution, a rotatory power between 0° and 15° and a sulphur content higher than 6%.

**EP 0 182 756 B1**

2. Cosmetic compositions according to claim 1, characterized in that the said hyaluronic acid has a molecular weight between 2.5 and 3 million.

3. Cosmetic compositions according to claim 1, characterized in that they contain, besides the said aqueous extract of umbilical cord and mucopolysaccharide complexes, also adjuvants and additives such as vasodilators, preservatives, wetting agents, fungicidal agents, surfactants, bactericides, vitamins, pH regulating agents.

**Claims for the Contracting State: AT**

1. A process for obtaining dermotropic cosmetic compositions comprising an aqueous extract of umbilical cord, characterized in that said extract of umbilical cord is free from sulphur and has a pH from 5 to 7, a kinematic viscosity at 30°C of $1 \times 10^{-1}$ cm$^2$xs$^{-1}$ to $2.5 \times 10^{-1}$ cm$^2$xs$^{-1}$ (between 10 and 25 centistokes), a hyaluronic acid titre ranging from 500 to 700 mg/dl and an ash content between 0.1 and 0.5%, is mixed in proportions ranging from 1:1 to 15:1 with further mucopolysaccharide complexes not containing proteins, having a pH from 5 to 7.5 in 5% aqueous solution, a rotatory power between 0° and 15° and a sulphur content higher than 6%.

2. The process according to claim 1, characterized in that the said hyaluronic acid has a molecular weight between 2.5 and 3 million.

3. The process according to claim 1, characterized in that the said aqueous extract of umbilical cord and mucopolysaccharide complexes are also mixed with adjuvants and additives such as vasodilators, preservatives, wetting agents, fungicidal agents, surfactants, bactericides, vitamins, pH regulating agents.

**Patentansprüche für die Vertragstaaten: BE CH DE FR GB LI LU NL**

1. Dermotrope kosmetische Zusammensetzungen umfassend einen wässerigen Nabelschnurextrakt, dadurch gekennzeichnet, daß der Nabelschnurextrakt schwefelfrei ist, einen pH-Wert von 5 bis 7, bei 30°C eine kinematische Viskosität von $1.10^{-1}$ cm$^2$.s$^{-1}$ bis $2,5.10^{-1}$ cm$^2$.s$^{-1}$ (zwischen 10 und 25 Centistokes), einen Hyaluronsäuretiter im Bereich von 500 bis 700 mg/dl und einen Aschegehalt zwischen 0,1 und 0,5% hat und im Verhältnis im Bereich von 1:1 bis 15:1 mit weiteren Mucopolysaccharidkomplexen gemischt ist, die keine Proteine enthalten, einen pH-Wert von 5 bis 7,5 in einer 5% wässerigen Lösung, eine optische Drehung zwischen 0° und 15° und einen Schwefelgehalt von mehr als 6% haben.

2. Kosmetische Zusammensetzungen nach Anspruch 1, dadurch gekennzeichnet, daß die Hyaluronsäure ein Molekulargewicht zwischen 2,5 und 3 Millionen hat.

3. Kosmetische Zusammensetzungen nach Anspruch 1, dadurch gekennzeichnet, daß sie außer dem wässrigen Nabelschnurextrakt und den Mucopolysaccharidkomplexen auch Adjuvantien und Zusatzstoffe, wie Vasodilatatoren, Konservierungsmittel, Netzmittel, Fungizide, grenzflächenaktive Agentien, Bakterizide, Vitamine, den pH-Wert regulierende Agentien enthalten.

**Patentansprüche für den die Vertragsstaat: AT**

1. Verfahren zur Gewinnung dermotroper kosmetischer Zusammensetzungen umfassend einen wässerigen Nabelschnurextrakt, dadurch gekennzeichnet, dass der Nabelschnurextrakt, der schwefelfrei ist, einen pH-Wert von 5 bis 7, bei 30°C eine kinematische Viskosität von $1.10^{-1}$ cm$^2$.s$^{-1}$ bis $2,5.10^{-1}$ cm$^2$.s$^{-1}$ (zwischen 10 und 25 Centistokes), einen Hyaluronsäuretiter im Bereich von 500 bis 700 mg/dl und einen Aschegehalt zwischen 0,1 und 0,5% hat, im Verhältnis im Bereich von 1:1 bis 15:1 mit weiteren Mucopolysaccharidkomplexen gemischt ist, die keine Proteine enthalten, einen pH-Wert von 5 bis 7,5 in einer 5% wässerigen lösung, eine optische Drehung zwischen 0° und 15° und einen Schwefelgehalt von mehr als 6% haben.

2. Verfahren nach Anspruch 1, dadurch gekennzeichnet, dass die Hyaluronsäure ein Molekulargewicht zwischen 2,5 und 3 Millionen hat.

3. Verfahren nach Anspruch 1, dadurch gekennzeichnet, dass der wässrige Nabelschnurextrakt und die Mucopolysaccharidkomplexe auch mt Adjuvantien und Zusatzstoffen, wie Vasodilatatoren, Konservierungsmitteln, Netzmitteln, Fungiziden, grenzflächenaktiven Agentien, Bakteriziden, Vitaminen, den pH-Wert regulierenden Agentien gemischt werden.

**Revendications pour les Etats contractants: BE CH DE FR GB LI LU NL**

1. Compositions cosmétiques dermotropiques comprenant un extrait aqueux de cordon ombilical, caractérisées en ce que ledit extrait de cordon ombilical est exempt de soufre, a un pH de 5 à 7, une viscosité cinématique à 30°C de $1.10^{-1}$cm$^2$.s$^{-1}$ à $2,5.10^{-1}$ cm$^2$.s$^1$ (entre 10 et 25 centistokes), un titre d'acide hyaluronique dans le domaine de 500 à 700 mg/dl et une teneur en cendres entre 0,1 et 0,5%, et est mélangé dans des proportions dans le domaine de 1:1 à 15:1 avec des complexes de mucopolysaccharides supplémentaires ne contenant pas de protéines, ayant un pH de 5 à 7,5 dans une solution aqueuse à 5%, un pouvoir rotatoire éntre 0° et 15° et une teneur en soufre plus élevée que 6%.

2. Compositions cosmétiques selon la revendication 1, caractérisées en ce que ledit acide hyaluronique a un poids moléculaire entre 2,5 et 3 millions.

3. Compositions cosmétiques selon la revendication 1, caractérisées en ce qu'elles contiennent en plus dudit extrait aqueux de cordon ombilical et des complexes de mucopolysaccharides, également des adjuvants et des additifs tels que des vasodilateurs, des agents préservateurs des agents fluidifiants, des agents fongicides, des tensioactifs, des bactéricides, des vitamines, des agents régulateurs de pH.

**Revendications pour l'Etat contractant: AT**

1. Procédé pour l'obtention de compositions cosmétiques dermotropiques comprenant un extrait aqueux de cordon ombilical, caractérisé en ce que ledit extrait de cordon ombilical est exempt de soufre, a un pH de 5 à 7, une viscosité cinématique à 30°C de $1.10^{-1} cm^2.s^{-1}$ à $2,5.10^{-1} cm^2.s^1$ (entre 10 et 25 centistokes), un titre d'acide hyaluronique dans le domaine de 500 à 700 mg/dl et une teneur en cendres entre 0,1 et 0,5%, est mélangé dans des proportions dans le domaine de 1:1 à 15:1 avec des complexes de mucopolysaccharides supplémentaires ne contenant pas de protéines, ayant un pH de 5 à 7,5 dans une solution aqueuse à 5%, un pouvoir rotatoire éntre 0° et 15° et une teneur en soufre plus élevée que 6%.

2. Procédé selon la revendication 1, caractérisé en ce que ledit acide hyaluronique, a un poids moléculaire entre 2,5 et 3 millions.

3. Procédé selon la revendication 1, caractérisé en ce que lesdits extrait aqueux de cordon ombilical et complexes de mucopolysaccharides sont melangés encore avec des adjuvants et des additifs tels que des vasodilatateurs, des agents préservateurs, des agents fluidifiants, des agents fongicides, des tensioactifs, des bactéricides, des vitamines, des agents régulateurs de pH.

Fig.1

Fig.2

EP 0 182 756 B1

Fig. 3

## Fig.4

GRAPH  1

EP 0 182 756 B1

Fig.5

GRAPH 2